# EUROPEAN PATENT SPECIFICATION

(11) **EP 4 051 006 B1**
(45) Date of publication and mention of the grant of the patent: **04.10.2023**
(21) Application number: 20824960.7
(22) Date of filing: 02.11.2020
(51) Int. Cl.: A01P 3/00, A01G 13/00, A01N 59/00, A61L 2/18

(54) **METHOD FOR ELIMINATING PATHOGENS IN PLANT GROWING AND CORRESPONDING SYSTEM**
VERFAHREN ZUR BESEITIGUNG VON KRANKHEITSERREGERN IM PFLANZENANBAU UND ENTSPRECHENDES SYSTEM
PROCÉDÉ POUR ÉLIMINER DES PATHOGÈNES DANS UNE CULTURE DE PLANTE ET SYSTÈME CORRESPONDANT

(30) Priority: 31.10.2019 FI 20195935
(43) Date of publication of application: 07.09.2022
(73) Proprietor: Happico Holding Oy, 05800 Hyvinkää (FI)
(72) Inventor: TAHVONEN, Risto, 21500 PIIKKIÖ (FI); KÄRKI, Kaija, 05800 HYVINKÄÄ (FI); VIERTO, Raine, 05800 HYVINKÄÄ (FI); LAPPI, Ari, 05830 HYVINKÄÄ (FI)
(74) Representative: Kespat Oy
(86) International application number: PCT/FI2020/050716
(87) International publication number: WO 2021/084164

(56) References cited:
- CN-A- 109 906 839
- JP-A- 2003 199 814
- US-B2- 6 817 541
- GARDONI D. ET AL: "Decay of Ozone in Water: A Review", OZONE: SCIENCE AND ENGINEERING., vol. 34, no. 4, 1 July 2012 (2012-07-01), pages 233-242, XP055780437, US ISSN: 0191-9512, DOI: 10.1080/01919512.2012.686354 Retrieved from the Internet: URL:https://www.tandfonline.com/doi/pdf/10 .1080/01919512.2012.686354?needAccess=true >

## Description

The object of the invention is a method for the elimination of pathogenic microbes from an object such as crops, the pathogenic microbes having stages sensitive to ozone during their life cycle, such as dried mycelia and spores, zoospores and sensitive mycelia that are unaccustomed to light. The method includes the production of an ozone water solution and the treatment of the object with the ozone water solution. A further object of the invention is a system for eliminating pathogenic microbes.

Ozone is an unstable gas consisting of three oxygen atoms. In nature, ozone only occurs in gaseous form as a component of the air, although its half-life is very short there as well. The highest ozone concentrations are found right at ground level, for example close to the surface of the grass on a clear summer day, whereby its concentration can reach a level as high as 80 ppb. Normally, ozone is not found close to the surface of the ground, as it is a molecule that decomposes quickly. When decomposing, the ozone molecule yields one oxygen molecule and one oxygen atom, the oxygen atom bonding with a corresponding oxygen atom to form an oxygen molecule or reacting immediately, for example, with a biological material. This reaction is a very quick oxidation reaction, whereby target biological material, for example a bacterium, dies immediately. Due to this oxidation reaction, ozone is one of the most efficient disinfectants and is used widely, for example, for water purification, air purification, whitening and disinfecting.

Ozone present in the air has been observed to control or slow down infections of pathogenic spores; however, the resulting control is inadequate from the standpoint of industrial production. Moreover, continuous exposure to ozone damages crops, as ozone in gaseous form in the composition of the air enters the inner parts of the plant leaf via the stomata. A constant exposure to a concentration of 80-100 ppb in the air already causes chlorosis in leaves, which impairs an efficient photosynthesis of the plant and thus reduces its potential yield. Consequently, the use of ozone in gaseous form for the elimination of pathogens is problematic and most frequently impossible in the situation of a development stage in which the pathogen takes the water it needs directly from the host plant or from the moisture surrounding the same.

The preparation of the ozone can comprise the dissolution of the ozone in water. The use of ozone dissolved in water in the destruction of crop pathogens is disclosed in US patent no. 6817541. This publication discloses technical solutions for the production of an ozone water solution and for its use in the elimination of crop pathogens in agriculture. The publication discloses an apparatus for the production of an ozone water solution, which includes a water tank and water pipe arrangement, an ozone generator as well as a Venturi pipe for feeding the ozone into the water pipe. The disclosed apparatus can be connected to any spraying system either in a stationary manner or on board a moving vehicle. The use of the ozone water solution for the elimination of harmful microbes such as bacteria, viruses and fungi from living crops is disclosed.

Patent publication CN 109 906 839 A discloses a system for growing plants indoor and for eliminating pathogenic microbes from crops using ozone water solution, which is sprayed on the crops using atomizing nozzles. Patent publication JP 2003 199814 A discloses a method of sterilizing rooms by using micro water droplets containing ozone, where the droplets are produced by ultrasonic atomization.

Known in and of itself is also the disinfection of growing substrates, irrigation water, irrigation pipes and irrigation channels between growing seasons using nitric acid and disinfectant.

High-pressure nozzles and turbulence-chamber nozzles are generally used in plant-disease and pest control for plant protection in this field. Gaseous ozone is quite poisonous at concentrations at which it is significant in plant-disease control. If ingested, the aqueous solution containing ozone is dangerous for man and animals.

The results in terms of plant protection achieved by treating crops with the ozone water solution according to the prior art vary considerably, although it has not been entirely clarified why.

The object of the invention is to provide an efficient, safe and completely non-toxic method for controlling plant pests with ozone that does not damage crops. In order to achieve this goal, the invention is characterized by the features disclosed in the feature part of the attached patent claim 1. A further object of the invention to provide a system by means of which ozone treatment can be carried out in a reproducible manner. The characteristic features of this system according to the invention are indicated in the attached patent claim 10 . A further object of the invention is to provide a method by means of which plant pests can be destroyed on the surfaces of crop processing spaces in a safe, efficient, non-toxic manner and generally by means of a single cleaning with ozone. The characteristic features of this method according to the invention are indicated in the attached patent claim 15.

The method according to the invention for eliminating pathogenic microbes from crops, the pathogenic microbes having stages sensitive to ozone over their life cycle, such as dried mycelia and spores, zoospores and sensitive mycelia that are unaccustomed to light, includes the production of an ozone water solution and the treatment of the crops with the ozone water solution. The evaporation rate is set so that the drying of the water on the surfaces of the crops takes 5 - 300, most advantageously 60 - 150 minutes. Potential, rare pockets of water are not a problem. A prolonged ozone treatment still kills all the pathogens. By means of the drying, the pathogenic spores and/or organisms in the crops are placed in a resting condition and an absorbent state before being treated with the ozone water solution. The ozone water treatment is carried out on the parts of the crops above ground with large droplets in such a manner that the ozone concentration of the ozone water solution on the surface of the crop to be treated is 100 - 3000 ppb, advantageously 200 - 1000 ppb, whereby the spores and/or organisms absorb the ozone together with the sprayed water and die.

In tests, the following standard time corresponding to a specific evaporation rate was obtained for the drying time in a greenhouse. At a relative humidity of 60 %, a radiation of 200 W/m² and an air flow of < 0.5 m/s, the required drying time is 1 - 7 hours, most preferably 2 - 4 hours. If there is a change in conditions and thus in the evaporation rate, a commensurate drying time is calculated to correspond to the same overall evaporation. In outdoor farming, the required drying times are shorter on account of the radiation of the sun and wind.

This way, pathogenic microbes can be eliminated from the surfaces of living plants without damaging ozone-sensitive plants, without any toxic residues and in a manner that is safe for the operator. By placing the pathogenic microbes in an absorbent state prior to the treatment with the ozone water solution, it is possible to ensure the efficacy of the ozone treatment, as the absorbent microbes are certain to absorb the water containing the ozone from their surroundings and die as a result of the oxidation reaction caused by the ozone. This improves the overall result in terms of the destruction of the pathogens by the ozone water treatment considerably.

In the method for eliminating pathogenic microbes from plant parts above ground in greenhouse farming, said plant parts comprising at least cucumber, lettuce and decorative plants sensitive to powdery mildew, wherein the life cycle of the microbes includes a significant stage outside the plant on plant parts above ground, the conditions of the plant environment are advantageously monitored, said monitoring comprising at least one measurement of the evaporation rate, by means of which measurement conditions are adapted, and a control unit controls the apparatus for spraying the ozone water solution in accordance with an adapted program.

This way, the ozone treatment can be carried out effectively in a greenhouse, which allows a completely non-toxic destruction of the pathogens. The efficacy of the ozone treatment is ensured by placing the pathogenic spores and/or organisms in an absorbent state before the ozone treatment and carrying out the ozone treatment according to a program installed on the control apparatus.

A surprising fact about ozone water should be noted in this connection. Even if the ozone concentration in unpressurized water is very high, it is no less than impossible to get the ozone back from the water in gaseous form as all of the ozone decomposes to oxygen when the water evaporates. This phenomenon creates the basis for the outstanding occupational safety of the method. The use of a corresponding ozone concentration in a greenhouse in gaseous form constitutes a challenging occupational safety risk and thus demands substantial provisions.

A further basis of the invention is the observation that ozone water cannot be sprayed onto plants with conventional high-pressure nozzles, which generate a small droplet size so that no ozone at all is detectable in the water that reaches the surface of the leaf.

Advantageously, in the method for eliminating pathogenic microbes in crops in outdoor farming, also comprising strawberry farming, wherein the plant pests spend a significant stage in their life cycle on the berry or plant parts above ground, the pathogenic spores and/or organisms are placed in an absorbent state by monitoring the ambient conditions, and the ozone treatment is carried out during the day at a time without rain. In outdoor farming, night-time dew keeps the plants wet and the plants are only dry at around 9 - 11. A greenhouse has humidity control and the plants are dry throughout the night.

The ozone treatment can be carried out effectively in outdoor farming this way, which permits a completely non-toxic destruction of the pathogens. The effectiveness of the ozone treatment is ensured by monitoring the evaporation rate, which in this case is a function of the temperature, humidity and flow speed of the air, and by carrying out the ozone treatment when the pathogenic spores and/or organisms are in an absorbent state.

Spraying nozzles can be used for the spraying of the ozone water solution, said spraying nozzles forming droplets the ozone concentration of which at the object is 10 - 75 % of its initial level in the water to be used for spraying. A sufficient ozone concentration is thereby obtained on the surface of the plant, as the ozone water solution is conveyed onto the surfaces of the plants in large droplets, which by their size are reminiscent of natural medium-intensity precipitation.

The initial level of the ozone concentration of the ozone water solution is 500 - 3000 ppb, advantageously 700 - 2500 ppb. The ozone concentration in the droplets on the surface of the plants is thereby sufficient for the elimination of pathogens.

Advantageously, in the method for destroying pathogenic microbes in a greenhouse or outdoors, the pathogenic microbes comprising at least grey mould, powdery mildew and downy mildew, the ozone water treatment is carried out at least once during germination and 0 - 6 times a day, advantageously 1 - 4 times a day, and at the very least once a week, as needed. It is thereby possible to effectively ward off spores having a short infection stage.

The daily allotment of the ozone treatments is advantageously determined based on a defined level of risk. It is thereby possible to optimize the daily number of sprayings, and by this means the efficacy of the ozone water treatment in the destruction of pathogenic microbes, based on infection models.

In the method for destroying pathogenic microbes in a greenhouse or outdoors, the pathogenic microbes comprising at least grey mould, powdery mildew and downy mildew, a single spraying of the ozone water solution can last 5 - 60 seconds, advantageously 10 - 20 seconds. The ozone exposure of sensitive plants thereby remains minimal.

The spraying pipe arrangement is advantageously emptied of the old water before the ozone water solution is conducted into the pipe arrangement. The ozone concentration of the ozone water solution is thus immediately at a sufficient level when spraying begins while the resting state of the microbes obtained by the drying is not wasted. In the worst case, the old water in the pipe arrangement saturates the microbes, whereby they are no longer sensitive to ozone.

Advantageously, clean growing mediums, clean biofiltered irrigation water as well as disinfected irrigation pipes and irrigation channels are provided when controlling Pythium disease in plants. The Pythium fungus is thereby destroyed before it has time to infect the crops. Pythium fungus is extremely sensitive to ozone water both as mycelium and as zoospores. Ozone has no effect on already infected plants, as the fungus is protected inside the plant.

The droplet size of the ozone water solution to be sprayed can be 0.5 - 10 mm, advantageously 1 - 8 mm on the Sauter mean diameter [SMD] scale. The ozone concentration of the droplets of ozone water solution that end up on the surfaces of the plants is thereby maintained at a level that is high enough for the destruction of the pathogenic microbes.

Advantageously, carbon dioxide is further added to the ozone water solution. The addition of carbon dioxide extends the half-life of the ozone significantly, whereby the ozone water solution is easier to store and it is easier to obtain a sufficiently high ozone concentration on the surfaces of the plants. The extension of the half-life results primarily from the increased acidity of the water solution brought about by the carbon dioxide.

Carbon dioxide can be added in such a manner that the pH of the ozone water solution decreases to a value of 5.0 - 6.9, advantageously to a value of 6.0 - 6.7. The half-life of the ozone thereby increases by a multiple of itself.

The system for eliminating pathogens from crops according to the invention includes an ozone-water solution generator for producing a 100 - 3000 ppb, advantageously 200 - 1000 ppb, ozone water solution, as well as a pipe arrangement for the conveyance of the ozone water solution comprising a trunk water pipe and several branch pipes ending in several spraying nozzles located among or above the plants. The system further includes observation means for monitoring the ambient conditions, comprising at least one sensor for measuring temperature and one sensor for measuring the saturation deficit. A control unit controls the apparatus for spraying the ozone water solution in accordance with an adapted program. Spraying nozzles are used for the spraying of the ozone water solution, said spraying nozzles forming large droplets in such a manner that the ozone concentration at the object is 10-75 % of its initial level.

A system by means of which the method described above can be carried out is thus realized. The advantages of the system are the same as the advantages of the method described above.

The ozone generator can be connectable via a valve to a standard fertilization/irrigation system. The system can thus be a part of a standard irrigation system by its trunk water pipe. There are, however, separate spraying pipe arrangements with separate spraying nozzles for the ozone water solution, as ozone decomposes immediately to oxygen in the air-humidification nozzles currently available in the sector. The system can thus include valves for selecting irrigation or an ozone water treatment as well as for draining the pipe arrangement.

The spraying nozzles for spraying the ozone water solution are advantageously low-pressure fan nozzles. It is thereby possible to spray the ozone water solution onto surfaces as large droplets, the ozone concentration of which fulfils the set criteria.

The method according to the invention for eliminating pathogenic microbes from the surfaces of crop processing spaces, the pathogenic microbes having stages sensitive to ozone during their life cycle, such as dried mycelia and spores, zoospores and sensitive mycelia that are unaccustomed to light, includes the production of an ozone water solution and the treatment of the surfaces with the ozone water solution. The evaporation rate is set in such a manner that the drying of the water on the surfaces takes 5 - 300 minutes. The pathogenic spores and/or organisms present on the surfaces are placed in a resting condition and in an absorbent state by the drying before the treatment with the ozone water. The ozone water treatment is carried out using large droplets in such a manner that the ozone concentration of the ozone water solution on the surface to be treated is 100 - 3000 ppb, advantageously 200 - 1000 ppb, whereby said spores and/or organisms absorb the ozone an die.

In outdoor farming, one waits for conditions in which the evaporation rate is sufficient to allow treatment after the drying stage.

For the first time, this creates the possibility of a completely non-toxic cleansing of the surfaces of growing spaces and stores. Moreover, the farming spaces can be used almost immediately after the ozone treatment. In the 1980's, the control of storage diseases of vegetables to be stored for a long period of time was achieved extremely effectively by spraying the exterior plant leaves located above ground with a fungicide. Entirely the same effect can also be realized by means of an ozone treatment.

In growing/storage spaces, the spraying of the ozone water solution can be performed with a manually operated spraying device. It is thus not necessary to install separate spraying systems in these spaces, as spraying can be performed cost-efficiently by means of the manually operated spraying device and the same device can be used in multiple spaces. Advantageously, in the method for eliminating pathogenic microbes from the surfaces of crop processing spaces, the surfaces are cleaned mechanically and dried so as to be air-dry, and 0.1 - 2 l/m², advantageously 0.5 - 1 l/m, of ozone water solution is used.

The invention is illustrated in the following in detail with reference to the attached drawings illustrating embodiments of the invention, wherein
- Figure 1: schematically illustrates a first embodiment of the system according to the invention for spraying ozone water solution for the elimination of pathogens in plant cultivation,
- Figure 2: illustrates the steps of the method according to the invention as a flowchart,
- Figure 3: schematically illustrates a second embodiment of the system according to the invention for spraying ozone water solution for the elimination of pathogens in plant cultivation.

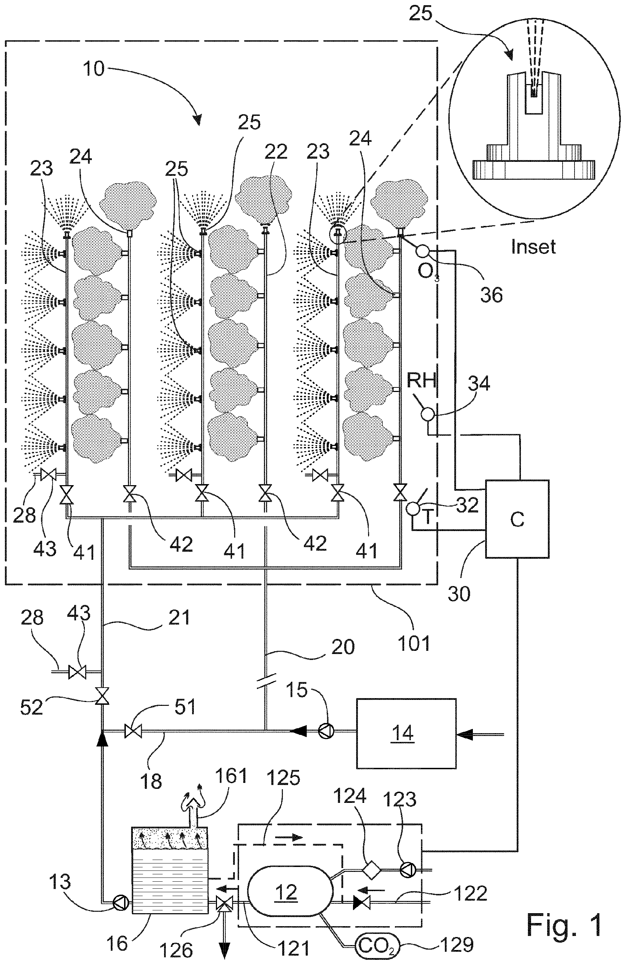

Figure 1 schematically illustrates an embodiment of a spraying system 10 according to the invention for the elimination of pathogens in plant cultivation using current agricultural technology. A typical irrigation-fertilization system of a greenhouse facility includes a mixing device 14 for mixing fertilizer and water and a pump 15, which are connected to a trunk water pipe 20. The trunk water pipe 20 branches out into several irrigation pipe arrangements 22, which are separated from the trunk water pipe 20 by magnetic valves 42 and which have watering nozzles 24 that are used for the dosed delivery of the plant fertilizer water to the plants.

In the embodiment illustrated in Figure 1, a stationary spraying system 10 is used for the dosed delivery of the ozone water solution to the plants. The greenhouse can be considered an enclosed space and is designated by the reference number 101. The production devices for producing the ozone water solution are located outside the greenhouse 101. The production devices include an ozone generator 12, a tank 16 and a pump 13, which pumps nearly pressureless ozone water into the trunk pipe 21.

The ozone water is produced in a known manner by feeding ozonated water under pressure (e.g. 4 bar) into lower-pressure water (2 bar) in the ozone generator 12. After a suitable delay, the ozone dissolves in the water and the ozonated water can be conveyed via a pressure-reducing valve 126 into a non-pressurized tank 16, where the ozone that is still gaseous is purged from the water and then from the tank 16 to the ambient air via an aeration valve 161. The remaining ozone water does not contain any gaseous part and can be pumped by the pump 13 into the low-pressure spraying system via the trunk pipe 21. No gaseous ozone is detected in the greenhouse 101 in spite of the use of water with a high ozone concentration for irrigation and pest control.

Carbon dioxide can be fed into the pressurized water at the same time as the ozone by way of the ozone generator 12. The carbon dioxide can be fed from a tank 129 into the pressurized water at the same time as the ozone by way of the ozone generator 12.

Beside the irrigation pipes 22 of the irrigation-fertilization system, there are also, among or above the plants, separate spraying pipes 23, which are separated from the ozone-water trunk pipe 21 by magnetic valves 41 and which have spraying nozzles 25 that are used for the spraying of the ozone water solution. The droplets of the ozone water solution to be sprayed can be adapted to have an optimal size by adapting the pressure of the ozone-water trunk pipe arrangement 21 and the size and type of the spraying nozzles 25.

Ozone is produced in the ozone generator 12 (for example, Prominent GmbH's (DE) model Ozonfilt OZVb 2A together with a separate compressor 123) from dry, pressurized air by corona discharge. The drying of the pressurized air is important so that the device includes an air dryer 124, advantageously an adsorption dryer. Humid air leads to the creation of nitric acid, which one tries to avoid. The ozone gas formed from the air is mixed into water, whereby a part of the ozone dissolves in the water and a part turns into small, undissolved bubbles in the water. The water is advantageously cool, approximately 15 °C. The ozone water solution is conducted from the ozone generator 12 via a hose 121 into the tank 16. Connected to the hose is a schematically illustrated pressure-reducing valve 126; however, the hose on its own and the dynamic pressure arising from the discharge of the ozone water from the hose 121 into the tank 16 bring about the requisite pressure reduction. When the pressure is reduced, the gaseous ozone separates immediately and evaporates from the water via the free fluid surface and via the ventilation pipe 161 to the ambient air. The ozone water to be pumped into the trunk pipe 21 by means of the pump 12 no longer contains gaseous ozone, but rather essentially all of its ozone is the ozone dissolved in the water.

The ozone concentration can be easily increased by circulating the ozone water along the line 125 into the raw-water feed pipe 122. This is in particular necessary when the temperature of the raw water rises, whereby the ozone's dissolution potential deteriorates.

When undissolved ozone evaporates into the air, the gaseous ozone can be detected by olfaction, which reacts as of a limit of approximately 11 ppb. The ozone generator 12 and the tank 16 are advantageously located outside the greenhouse. The undissolved ozone in the water thereby evaporates into the air before the ozone water solution is conducted into the greenhouse, where it is not necessary to take any additional measures with respect to occupational safety. The use of the ozone water solution in accordance with the described method in the control of pathogens is consequently safe for an operator, as absolutely no ozone gas enters the air of the greenhouse during the spraying of the ozone water solution.

The use of the tank 16, however, is not imperative from the standpoint of the implementation of the invention, but it increases occupational safety in narrow spaces. The ozone water solution can also be drawn directly from the valve 126 (three-way valve) for use in the spray. The use of an ozone generator 12 with a low output (producing a small amount of ozone) relative to the air volume of the greenhouse 101 inside a greenhouse 101 with a large volume does not necessarily compromise occupational safety. It is in any event advantageous to guide the air-conditioning pipe 161 of the tank 16 to the ambient air, whereby the apparatus can be used in complete safety in enclosed technical spaces of the greenhouse.

The control of the spraying of the ozone water is advantageously carried out by a control unit 30 of the irrigation-fertilization system, which monitors the temperature of the area with a sensor 32 and the saturation deficit (relative humidity RH) with a sensor 34, which indicates the state of humidity of the plants and pathogens. An ozone concentration sensor 36 can further be added to the system, which measures the ozone concentration of the ozone water solution at the very end of an irrigation pipe arrangement 22. The control unit 30 controls all valves of the system. A single magnetic valve 41 can control the spraying of an area of 250 - 500 m² depending on the nozzle, the output of the ozone facility and the model of the nozzles.

By way of example, ProMinent's electrochemical ozone sensor DULCOTEST (OZE 3-mA-2 ppm), the measuring range of which is 0.02-2.0 mg/l, can be used for the real-time measurement of the ozone concentration of the ozone water solution. For the analysis of the ozone concentration of an ozone water sample and the calibration of the electrochemical sensor, for example, ProMinent's photometer (DT1B), the measuring range of which is 0.03-4.0 mg/l, can be used.

When the ozone generator 12 is on stand-by, the ozone water solution present in the spraying pipes 23 and the trunk pipe 21, in which solution not enough ozone remains for disinfection until the next spraying, can be removed via drain valves 43 and drain pipes 28 in communication with the magnetic valves 41 in the spraying pipes 23 and the valve 52 in the trunk pipe 21. When the ozone generator 12 is switched on, the magnetic valves 41 at the extreme end of the trunk pipe 21 open until the trunk pipe 21 is full, following which the spraying pipes 23 in turn spray large water droplets onto the plants via their spraying nozzles 25. Depending on the leaf surface area of the plants, 1 - 4 dl/m² of ozone water is advantageously used at a time.

The surfaces of the plants are wetted with the ozone water solution during the day by daylight so that the surfaces of the plants are sure to be dry and the pathogens in an absorbent state prior to treatment. The daily number of instances of wetting is determined in accordance with a risk level based on infection models, wherein:
- Risk level 0: is a resistant variety,
- Risk level 1: is a pest-tolerant variety and no symptoms are observed in the plants,
- Risk level 2: is a susceptible plant and pests are not yet detected in the plants, and
- Risk level 3: plant pests are detected in the plants.

The spraying of the ozone water is alotted based on the risk levels, wherein:
- Risk level 0: consists in 0 sprayings / day,
- Risk level 1: consists in 1 spraying / day,
- Risk level 2: consists in 2 - 3 sprayings / day, and
- Risk level 3: consists in 3 - 4 sprayings / day.

The spraying nozzles currently used in the control of diseases and pests are by type high-pressure nozzles or turbulence-chamber nozzles, which are not at all suitable for ozone treatment, as the ozone in them decomposes immediately to oxygen. If the ozone water solution is sprayed using these spraying nozzles, no ozone at all reaches the object, as the ozone dissipates entirely from the water solution during the formation of the drop. When the ozone concentration of the water was 800 ppb and the ozone water solution was sprayed in small nebulized drops into a measuring bottle, the ozone had already entirely disappeared in first measurements. However, when ozone water with a concentration of 800 ppb was sprayed in large droplets, the ozone concentration in the measuring bottle was 340 ppb. When ozone water was sprayed in large droplets onto a test object, the result was extremely lethal for the spores of powdery mildew, for dry grey mould and for Fusarium.

A requirement of the control treatment is that the ozone water solution must be conveyed to the surface of the plant in large droplets, which by their size are reminiscent of natural medium-intensity precipitation. Fan nozzles can be used as the spraying nozzles 25, for example a white (product code: NNA003108) or black (product code: NNA003110) 110° fan nozzle, at a low pressure of 100 - 200 kPa. The flow rate of the ozone water solution is thus 1.85 - 3.26 l/min, while the ozone concentration of the droplets that reach the surfaces of the plants is 10 - 75 % of the initial level in the water to be used for spraying.

By means of this treatment, at least powdery mildew, downy mildew (*Pseudoperonospora cupensis* on cucumber, *Bremia lactuce* on lettuce), grey mould, black stem rot of cucumber (*Didymella bryonie*) and the dry spores awaiting moisture of stem canker of tomato (*Didymella lycopersici*) are cleansed from the surfaces of plants. By means of the same treatment, pests such as spider mites and insects with sensitive skin are reduced or controlled. By means of the same technique, it is possible to ward off powdery mildew, grey mould and spider mites from outdoor strawberry plants by using the spraying devices available for cooling and frost protection.

The wetting of the plants with ozone water solution can hardly increase measurable values of the ozone concentration in the air, as the amount of ozone water per leaf surface area is extremely small. The leaf surface area index for cucumber plants is typically 3 - 5, i.e. one hectare has 3 - 5 hectares of leaves, for the even, one-time wetting of which approx. 400 l - 2000 l of water is needed in the plant-protection spray. The amount of ozone based on 400 litres is approx. 0.32 g/ha when the ozone concentration in the water to be sprayed is 800 ppb and assuming that all the ozone is transferred without dissipating into the air. The hectare volume of a modern greenhouse is approx. 70 000 m³.

Ozone probably dissociates from the water sprayed onto the leaf in less than 10 minutes, but this requires more precise measurements or an estimate that can be performed computationally. For example, ozone dissipates from water sprayed in a fine spray immediately and before it reaches the leaf. It can be stated with certainty that it is possible to spray a droplet layer of ozonated water, the initial concentration of which is 1000 ppb, onto plant surfaces. For plants with a high ozone tolerance, the concentration should probably be significantly higher, as the surface layer of the plant probably also acts as a catalyst, decomposing the ozone as soon as it comes into contact, for example, with the waxy cuticle. An inorganic material known to have a corresponding catalytic effect is, for example, brass. An ozone concentration that is too high, however, can be noxious to the plants. With the described method for producing ozone water solution, wherein ozone is produced from dry air, a harmful concentration of ozone cannot reach the plants. The optimization of the ozone concentration of the ozone water solution is crucial from the point of view of both an effective destruction of the pathogens as well as the avoidance of any jeopardization of occupational safety or damaging of the plants.

The single-celled root hairs of the young roots of the plants, by means of which the plant gets the plant nutrients it needs, are probably extremely sensitive to ozone, but the ozone has no chance of penetrating the root system of the plant, as it decomposes immediately upon encountering the organic material in the soil. In preliminary tests, a concentration of approximately 800 ppb in the water did not cause any damage to the root system of cucumber or any weakening of the plant when the stem bottoms of the plants were watered twice a week and the plants were evaluated a week after the last treatment.

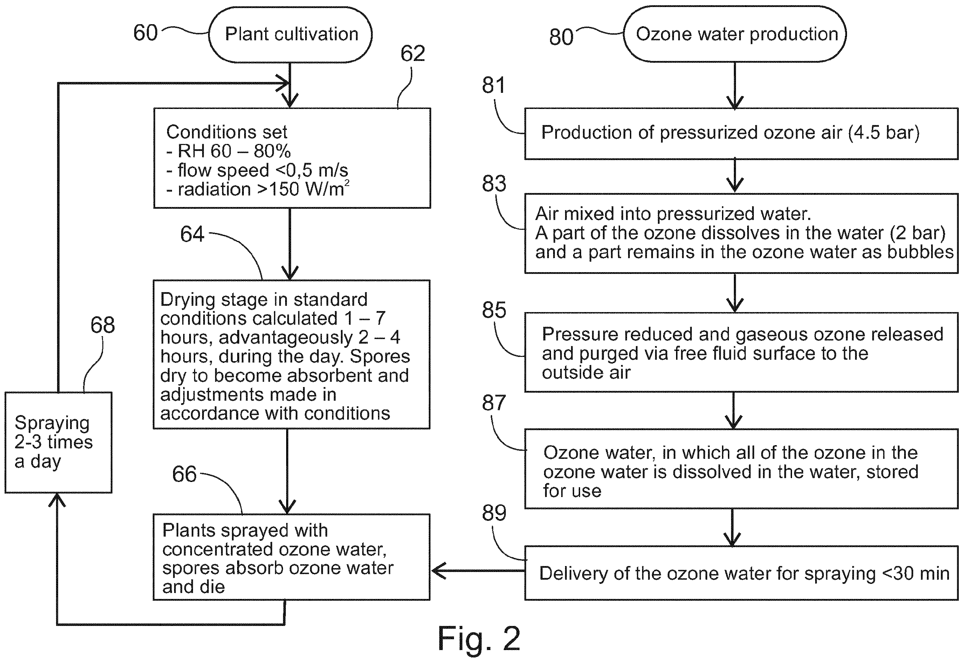

An example of a plant pest control according to the invention is illustrated in Figure 2. The cultivation of the plants 60 and the ozone water production 80 each constitute independent sub-processes. In the cultivation of the plants, optimal growing conditions are maintained in the greenhouse, step 62, typically exhibiting the following values:
- relative humidity RH 60 - 80%
- flow speed <0.5 m/s
- radiation level 200 -400 W/m²

The radiation level, advantageously 150 - 800 W/m², naturally fluctuates, but a sufficient radiation is obtained by the greenhouse lamps, approximately 250 W/m², if sunlight is not available. In particular the radiation has a strong influence on the microclimate, in which the pathogenic microbes live, occurring on the surface of the leaves. Without these external factors (radiation and air flow), the saturation deficit of the microclimate falls to zero even if the relative humidity RH in the greenhouse is significantly below 100 %.

In outdoor farming, "adaptation" means observing and waiting for conditions that bring about a drying action. The spraying according to the invention is carried out if conditions permit. The level of radiation generated by the sun frequently surpasses the optimal value from the standpoint of growth.

From the standpoint of the invention, the drying step, 64, is essential. In normal conditions, the drying step takes 1 - 7 hours, most advantageously 2 - 4 hours, during the day, whereby spores dry out so as to become capable of absorption. At night, the plants are not watered; besides, the spores do not spread in the damp plants with the wind at night.

After the drying step, the spraying of the plants occurs with the concentrated ozone water, step 66, whereby the spores absorb the ozone water and die.

Spraying is repeated 2 - 3 times a day, step 68.

The production of the concentrated ozone water, 80, can be carried out, for example, as follows.

In a first step, pressurized ozone air, step 81, (typically 4.5 bar), is produced and dried.

The obtained pressurized ozone air is mixed, step 83, into pressurized water (2 bar) and ozone water is produced, in which a part of the ozone dissolves in the water and a part is in gaseous form.

The pressure of the ozone water is then reduced, step 85, whereby the dissolved ozone exceeding the saturation limit and the gaseous ozone are quickly released from the tank via the free fluid surface to the outside air.

The "pure" ozone water, in which all of the ozone is dissolved in the water, is stored for use, step 87. The dissolved ozone decomposes quite slowly to oxygen, whereby the stored ozone water can be used effectively for 30 minutes without carbon dioxide and even several hours with carbon dioxide.

Finally, this concentrated ozone water is provided for spraying, step 89.

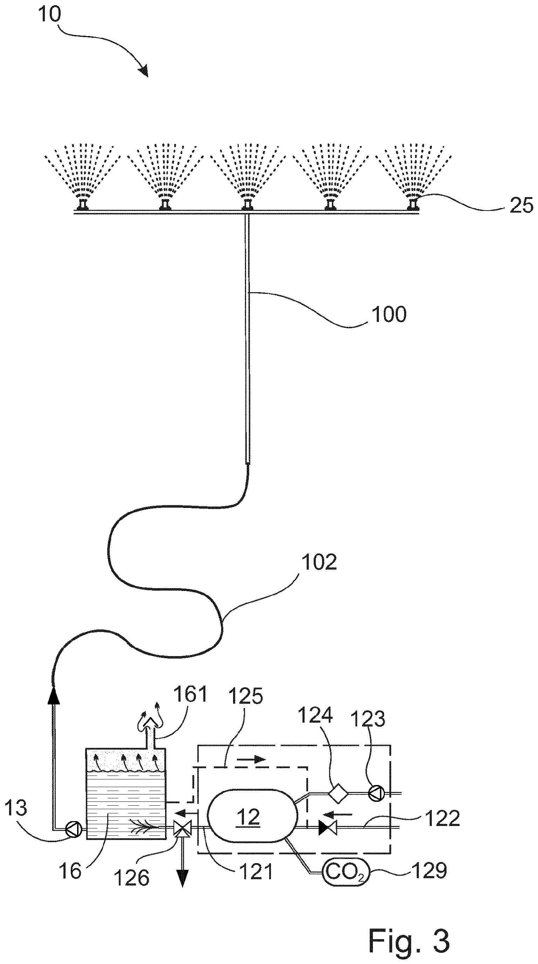

Figure 3 schematically shows a second embodiment of the spraying system 10 according to the invention, which can be used for eliminating pathogens from crops as well as from greenhouse structures and stores such as, for example, vegetable, fruit and potato stores. In this embodiment, the ozone treatment is performed with a manually operated disinfecting pole 100, which can be used, for example, between growing seasons or for periodic treatments during growing. The same ozone generator 12, tank 16 and pump 13 as in the embodiment shown in Figure 1 can be used in this system. The ozone water solution is conducted from the ozone generator 12, tank 16 and pump 13 via a flexible hose 102 to the disinfecting pole 100 operated by a person, the disinfecting pole 100 having spraying nozzles 25 over a width of, for example, 2 - 4 metres. The manually operated disinfecting pole 100 can also be utilized for the treatment of plants as described above. When cleaning, as with crop growing, a corresponding drying step must always be carried out.

After the mechanical cleaning of the production spaces between growing and storage seasons, the spaces are left to dry thoroughly so as to be air dry. A wetting layer of 1 - 3 dl/m² of concentrated ozone water solution is sprayed onto the surfaces to be cleaned by means of the specially structured disinfecting pole 100. Ventilation openings and doors are open during treatment. In less than two hours, the ozone decomposes to oxygen in the water, whereby all pathogenic fungi, bacteria and viruses in a resting state are also destroyed.

By means of a corresponding ozone water treatment, it is also possible to clean working implements such as tractors, which are used at numerous different sites, easily and effectively. This way, it is possible to effectively reduce the transfer of plant pests from one growing area to another.

### Ozone and plant pathogens

Ozone can only have an effect during cultivation on plant diseases whose life cycles include a significant susceptible stage in plant parts above ground on the outside of the plant. The use of ozone in the control of plant diseases consequently requires that the treatments are performed before the pathogen penetrates the interior of the plant. Perhaps the most surprising, yet most significant circumstance influencing control success is the fluctuation of the properties of different types of pathogens with respect to their vulnerability to ozone and in different stages of development. This fluctuation is also decisively influenced by the appearance of the disease in plant parts above or below ground.

Plant diseases are caused by the following three groups of organisms: fungi, bacteria and viruses. Fungal diseases are the largest, most diverse and economically most significant group, which cause over 80 % of the total damages caused by diseases in Finland, depending substantially on the plant species.

### Viral diseases

Most viral diseases are spread by way of insect vectors, a typical example being the aphid, i.e. the insect sucks liquid from a sick plant and spreads the virus particles present in the liquid to the next plant. As a result of this manner of dissemination, there is no opportunity for ozone to interrupt the dissemination paths. Another dissemination path is asexually propagating plants. There is no opportunity here either for ozone to have an impact on this manner of dissemination. Some viruses remain for long periods of time in a resting state in growing substrates. When the greenhouse is disinfected for other reasons, the virus is killed quickly by the effect of the ozone.

### Bacteria

Bacterial diseases, which remain on the surfaces of agricultural structures and in product stores from one growing season to the next, can be controlled easily with ozone water as a side effect when the structures are cleansed of fungal diseases. It is very well known that ozone thoroughly kills bacteria present in water and in structures regardless of the species.

### Fungal diseases

The use of ozone in the control of fungal diseases requires a thorough knowledge of the epidemiology of different fungal diseases, their physical structure from a control standpoint and the preconditions of an infection in different groups of fungal diseases.

A significant new discovery regards plant diseases the mycelium and spores of which are continually susceptible to the light of the sun during growth. During growth, the fungus takes water and nutrients from its host plant and its organic functions are completely active, whereby they are at full fluid tension. Ozone in gaseous form or in liquid form has no effect on this stage. An illustrative disease, common grey mould, is entirely resistant to ozone in very high concentrations. Another good example of a fungal mycelium that grows in light is the mycelium of powdery mildew, which grows as a very thin, light-coloured mycelium on the upper surface of the plant leaf. The fungus grows haustoria through the epidermis by means of which which it takes fluids from the host plant for its nourishment. This part of the powdery mildew withstands water, aridity, sunlight and ozone in both states even in high concentrations. However, prior to infection, the spores of the fungus and the germ tube growing from them are sensitive to ozone both in gaseous form as well as especially in water.

Another significant discovery is the vulnerability of fungi growing in the soil to ozonated water also in a stage of active growth. Testing has now been carried out with Pythium and Fusarium.

### Powdery mildews (Erysiphaceae)

After infecting the leaves, powdery mildews form a thin mycelium on the surface of the plant, which gets the moisture and nutrients it needs through the surface of the leaf directly by means of its haustoria. The mycelium forms its light-coloured conidia asexually and abundantly on the surface of the plant, which the wind transports over large distances to new plants. The pathogen is an obligate parasite, i.e. it only manages to grow by means of living plants. Almost every plant has its own species of powdery mildew or at least its own race of a species, which cannot make another plant species ill like it does its own.

The spores of powdery mildew have an exceptionally high moisture content compared to the spores of other fungal diseases, which is why merely a very high air humidity suffices for a spore infection, yet the conidium is destroyed quickly when engulfed by a water droplet. The spores of the fungus spread during the day and make the plant ill at night. Depending on the species of the powdery mildew, the infection stage takes 6 - 12 hours. In gaseous form, ozone has a satisfactory effect on the infection with constant exposure, while the ozone solution yields a good or excellent control result when the plants are sprayed three times during the day with water droplets containing over 300 ppb of ozone upon contact with the surface of the plant. Ozone does not destroy the mycelium present on the surface of the plant so that it is not possible to cleanse the leaf of the plant of the hyphae of the fungus with ozone. Using this knowledge, it is possible to construct technical applications for implementation in greenhouses as well as outdoors for the control of powdery mildew disease infections. Based on this solution, many other plant pests which spread first to the surfaces of the plants as dry spores that have acclimated to the shining of the sun during their evolution (for example grey mould) can also be controlled.

### Downy mildews

Downy mildews (*Peronosporaceae*) are obligate parasites, which only live by means of a living plant. The fungus grows a mycelium inside the plant and simultaneously destroys the plant's cell tissue by means of its secretions. In the zone of propagation, conidiophores emerge from the stomata. Conidia spread with the wind to new plants. An infection is only successful if there are water droplets on the surface of the plant in which the spores release zoospores that swim in water, which penetrate the interior of the plant through its natural apertures such as the guttation points at the tips of the leaves and the stomata. An infection can occur in an hour and normally takes two-three hours.

Downy mildews cause diseases on cucumber, lettuce and roses. The most serious diseases occur on cucumber and lettuce by means of which the plants can be entirely destroyed as quickly as within a week. Control measures are an efficient adaptation of the climate by heating and ventilating. The use of ozone water in connection with the control of powdery mildew is without a doubt effective on the disease, as the zoospores are extremely sensitive to ozone, as is the case with all corresponding Oomycetes fungi forming zoospores.

### Soil-borne Pythium water moulds (Pythiaceae)

Pythium-type pathogens are all soil-borne diseases which cause damping off and root rot. The fungal mycelium can also live and remain in the soil as a saprophyte in organic material. The species *Pythium ultimum* occurring in greenhouses hardly ever forms sexual oospores in the manner of P. *depaeryanum,* which commonly occurs in field farming. The fungus forms colonies in the rapidly growing mycelium, from which zoospores are released into the soil water, the zoospores first infecting the root via the root hairs at the tips of the roots and then moving on to the hypocotyl, causing death in seedlings and root decay and stem disease in older plants. The most significant manner of dissemination of this disease is a contaminated plant substrate and recycled irrigation water. Pythium disease is currently the most significant disease of root systems in greenhouses especially on cucumber and potted lettuce.

Pythium is extremely sensitive both as mycelium and zoospores to ozone water. Ozone has no effect on plants that are already ill, as the fungus is protected inside the plant and the growing medium surrounding the roots provides a perfect protection from the ozone. Consequently, a control and limiting of Pythium disease is grounded in clean growing mediums, clean biofiltered irrigation water and the disinfection of irrigation pipes and irrigation channels.

As Pythium is extremely sensitive to ozonated water, the use of ozone is a very fast and efficacious measure in the cleaning of empty growing spaces and irrigation systems. An advantage over other disinfecting measures is further the ease, safety and speed of the treatment.

### Fusarium and other soil-borne imperfect fungi (fungi imper-fecti) with a corresponding manner of dissemination

A numerous set of soil-borne fungal diseases, which damage the roots, stems and vascular bundles of the stalks of the plants, spread in the growing medium used. The most widely-known and most significant fungus species is *Fusarium oxysporum*, of which there are shape species specializing in different plants, which cause wilt diseases as the vascular bundles inside the stalks of the plants are destroyed. The fungus advances in the vascular bundles to the leaves, on the lower surfaces of which the fungus forms small 1-3-celled airborne spores, which, once dried, can survive in the structures for long periods of time. Adhesive mycelium and spores also remain in the greenhouse in the plant substrate and in old root fragments. When greenhouses are emptied at the end of a growing season and the remains of the plants and plant substrates are cleaned up, small fragments of the plants and the plant substrates always remain in the greenhouse, the disinfecting of which is essential before the introduction of new plants.

All pathogenic fungi which go into a lengthy resting state as a consequence of drying start to grow again as soon as they absorb water. When the ozone water for wetting is sprayed onto these dry surfaces, the spores and mycelium fragments as well as the dormancy forms (chlamydospores) of the fungi absorb this water. As these pathogens were never able to develop an internal resistance to ozone during their evolution, they are destroyed immediately. Fusarium fungi are also susceptible when fully turgid unlike fungal hyphae that grow continuously with sunlight (for example powdery mildew and grey mould).

### Grey mould (Botrytis cinerea) and other fungal diseases which have learned to endure and even utilize sunlight and large fluctuations in humidity in their dissemination

Grey mould turns out to be surprising against ozone. When grey mould was washed from the surface of stored cabbage that had been rotting for an extended period into water and sprayed back onto healthy cabbage, plastic, glass and the severed surface of a redcurrant branch, an ozone concentration of 2500 ppb in the air was not able to kill off the spores and mycelium of the grey mould. When test set-ups yielded the same result the following year, in which grey mould spores and the associated mycelium fragments washed from the surface of moist, rotting cabbage survived on a plastic surface in an ozone spray and in a concentrated ozone solution, it was completely clear that the surfaces of the mycelium and spores of grey mould are completely resistant to ozone. When a corresponding test was performed with a fungal suspension washed from a plate of dried grey mould, the grey mould died almost completely. This long test series showed that a disinfection of plant surfaces with ozone water must be performed in conditions in which the spores that reach the surfaces of the plants are so dry that they start to absorb water in order to start to grow and infect the plant. At the same time, this test series showed that grey mould does not possess a mechanism by means of which it could prevent the penetration of the interior of the cells by the ozone in the water.

In nature, grey mould survives until the following year on the surfaces of rotten plants such as berries and succulent leaves, where they form conidia in conidiophores in moist conditions. In wet conditions, the spores hardly spread with the wind; however, once dried by the sun, the spores of the growth break free to be carried a distance of at least 50 metres by the wind if the wind speed exceeds 2 m/s. Dried mycelium and conidiophores are readily identifiable under a microscope by their structure compared with completely turgid growths.

The preservation of the spores of all fungal diseases of the grey-mould type in nature is the same when they are dry and their germination works by means of the same technique, i.e. dry spores absorb water from their surroundings by means of an osmotic pressure difference and then begin to grow. For the control of these fungi, there is consequently only one efficacious method using ozone water, i.e. the plants and the inactive surfaces to be cleaned are treated when they are as dry as possible. It is very likely that, during the evolution of fungi, populations were selected to continue their activity whose mycelium and especially whose spores facilitated by means of their surface-tension characteristics the binding of the water to the surface of the spore in the most efficacious manner so as to accelerate germination. When ozone water is sprayed onto the surface of the plant, an individual droplet breaks up into small droplets as a result of its mechanical force to efficiently wet the fungal spores that are present and the surface of the plant, wherein the water very quickly accumulates to again form large droplets as a result of the surface tension of the leaf of the plant. This physical motion of the water helps to efficiently wet the fungi with ozone water.

In 2019, extensive foundational research was conducted on the impact of ozone water on the main fungal diseases of greenhouse plants, powdery mildew, wilt disease, damping off and grey mould (*Erysiphe sp.*, *Fusarium oxysporum*, *Pythium ultimum* and *Botrytis cinerea*). The central results of this research were the following:
1. The soil-borne and water-borne *Pythium* fungus was sensitive to ozone, but its control in the soil was not possible with ozone, as ozone decomposes immediately in the soil.
2. The spores of powdery mildew are extremely sensitive to ozone, but the mycelium of the fungus growing on the surface of the plant survives.
3. The spores and mycelium of the Fusarium fungus are extremely sensitive to ozonated water both in a solution and in sprays on the surfaces of dry materials.
4. The mycelium and spores of grey mould taken from wet cabbage are ozone-resistant, but its dried mycelium and spores are sensitive to ozone. This fungus only spreads to plants as dry spores.
5. Not even highly concentrated ozone water causes problems of toxicity for the leaves of sensitive plants as a spray or when watered to the roots.
6. The technique using ozone water plays a decisive role in the control of fungal diseases. Susceptible microbes die within a few minutes in ozonated water. However, if ozone water is sprayed onto the surfaces of plants and fixed structures by means of conventional spraying techniques used in the control of plant diseases and pests, the ozone decomposes to oxygen entirely during the spray's journey from the nozzle to the surface to be treated and consequently completely loses its effect. For this reason, special nozzles must be used in the sprayings, said nozzles forming droplets the ozone concentration of which at the object is approximately half its initial level.

The research results described above regarding the effective mechanisms of ozone on the epidemiology of different groups of plant diseases yielded a nearly perfect blueprint for modelling the use of ozone water in plant protection, as it was already known from other sources and has been known for decades that bacteria and viruses are completely sensitive to ozone present in water, that water is absorbed through the skin of a spider mite and, as an example of an insect, that the soft skin of the thrip is very sensitive to high ozone concentrations in water.

The test results are explained in greater detail in the following.

### Examples and test results

### Tests regarding control of cucumber powdery mildew

### Preliminary test March-April 2019

Mildewed cucumber leaves were collected from a garden adjacent to the research unit with which 6 outdoor cucumber seedlings were infected. After the verification of the manifestation of an infection, 18 new seedlings were grown up to the stage of the first plant leaf, at which point the plants were divided in onion boxes into 3 groups in the middle of each of which 2 ill plants were placed. The control plants were sprayed three times a week with water. The second test group was sprayed three times a day with ozone water (800 ppb) and the third test group was sprayed mornings and evenings. The sprayings were carried out with large droplets, which remained on the surface of the plant 2 - 4 hours. At night, the plants were covered with a monochrome plastic in order to maintain a high humidity. The sprayings took place over 6 weeks.

This test showed that it was possible to limit the spread and intensity of the powdery mildew despite the fact that the infected plants were in the immediate vicinity of the test plants. In the most intense treatment, the powdery mildew infection of the infected plants was also reduced. A very important discovery was that the spraying of the ozone water onto the plants did not cause leaf symptoms on the cucumber leaves, which were typical in earlier tests conducted with gas, in any of the treatments.

### Actual test April-May 2019

While the preliminary test was still underway, 20 cucumber seedlings were grown to the stage of a single leaf, the seedlings then being divided up into 5 boxes. The test groups were
1. entirely untreated
2. water spraying three times a week and watering of pot with ozone water for 3 and for 2 weeks before the conclusion of the test
3. ozone spraying 3 three times a week
4. ozone spraying 5 times a week at 7 a.m. and 4 p.m.
5. ozone spraying 5 times a week at 7 a.m., 12 p.m. and 4 p.m.

The test area, in which mesh-bottomed pots were planted in peat with 4 pots in each box, was surrounded with the sick seedlings of the preliminary test, which were at a distance of approx. 60 cm from the test plants.

The ozone sprayings five times a week 2 or 3 times a day significantly reduced the powdery mildew infection of cotyledons and plant leaves over the entire test. Halfway through the test, all of the plant leaves were healthy and the cotyledons only had a slight infection. The treatment conducted three times a week reduced the infection right up until the end of the test. In a microscopic examination, a very fragile-looking mycelium growing on the surface of the plant, which sucks water and nutrients from the plant by means of its haustoria, and which in nature is also always susceptible to the sun and an ozone concentration as low as 80 ppb, completely withstands the now applied ozone concentration of 340 ppb of the spraying. The conidia of the disease are evidently very susceptible to ozone water, whereby a daily spraying is sufficient to prevent an outbreak of new diseases.

The ozone sprayings did not damage the leaves of the plants at any stage. The watering of the roots of the plants did not have an impact on the development of its fresh weight. The roots of the plants were also undamaged compared with the untreated test group.

### Laboratory tests with fungal diseases

### Fusarium oxysporum tests

### Test 1. 9.4.2019

A plateful of *F. oxyxsporum* fungus without agar was homogenized with 100 ml of water. 10 ml and 1 ml of the fungal suspension were added to 100 ml of sterile water and 100 ml of 800 ppb ozone water. After 10 minutes, the inoculums from each treatment were placed in 3 PDA plates.

Observation 15.4.: culture colonies: in both water-treatment plates, >100 colonies/plate. In the ozone treatment, 8 - 17 colonies in the plate with 10 ml of inoculum and 1 - 3 colonies in the plate with 1 ml of inoculum.

### Test 2. 16.4.2019

A plateful of *F. oxysporum* fungus was homogenized with 100 ml, which was sprayed onto a plastic surface. After 16 hours of drying, ozone water was sprayed onto the plastic in large droplets. Once the droplets had dried, the plastic was cut into 4 pieces for an agar medium substrate in order to ascertain with three replicates the vitality of the fungus.

The dried *Fusarium* fungus on the plastic surface died completely when it was sprayed with large droplets on the plastic and the water was left to dry after treatment.

### Grey mould tests (Botrytis cinerea)

### Test 1. 10.4.2019

Grey mould spores rinsed from cabbage were homogenized in approximately 200 ml of water. 10 ml and 1 ml of this fungal suspension were added to 100 ml of water and 100 ml of ozone water (800 ppb). After 10 and 60 minutes, specimens from each treatment were placed into 3 PDA plates.

Observation 15.4: in all plate specimens, in addition to B.c, other microbes were so abundant that the colonies could not be counted, i.e. the result was that the ozone water did not reduce microbiota.

### Test 2. 16.4.2019

A petri dish, (a CMA plate, in which cabbage discs stimulate the formation of conidia), comprising the almost dry old fungal growth and the endophytic bacteria from the cabbage therein), grey mould spores were rinsed into 100 ml of water. 1 and 10 ml of this water were pipetted into 100 ml of water and 100 ml of ozone water. 0.5 ml of each of these water mixtures was pipetted into 3 petri dishes in order to determine the vitality of the grey mould.

After a week of cultivation, it was observed that the ozone water effectively killed the grey mould and the bacteria present in the solution in both concentrations of the fungus.

### Pythium ultimum tests

*Pythium ultimum* differs by its structure and by its biology significantly from the other fungal diseases to be tested. The fungus grows a septate-less mycelium very quickly in soil containing humus such as steamed peat and an artificial substrate in a laboratory so as to fill a 9 cm dish within 24 hours. In the soil, the fungus first colonizes the young root hairs at the tips of the plant roots by means of which the plant takes nutrients from the soil. Colonies quickly spring up within the mycelium in the root hairs, from which colonies tinsellated zoospores are released. The zoospores swim in the soil water, thus spreading the disease to new objects. In circulating water, the zoospores are quickly conveyed with the irrigation water over an extensive area if the irrigation water is not cleansed of the pathogen. The Pythium fungus is limited on comestible crops by means of rigorous hygiene and biological control and filtered irrigation water. After farming, the growing mediums and irrigation devices are disinfected.

### Test 1. 23.5.2019.

In a first dish, a fungal culture was soaked for 30 minutes with sterile water when the Pythium fungus had grown to a distance of approx. 2 cm away from the small piece of inoculating agar at the rim of the dish, which was removed with a large cork borer before the water treatment. The fungus grew to fill the dish within 16 hours during the water treatment. In a second dish, an identical treatment with ozone water (800 ppb) was carried out. A thicker fungal mycelium was visible in the Pythium culture around the site of inoculation, from which the fungus only had time to grow 2 cm before the ozone treatment. The ozone destroyed the youngest tips of the hyphae of the fungus and the hyphae that had grown apart from the agar, but had no effect on hyphae that had already grown partially into the agar.

The ozone treatment destroyed the hyphae on the surface of the agar. When Pythium grows on an artificial substrate, a part of the mycelium grows into the agar in the same way that a pathogen penetrates in a unified front succulent plant parts such as the hypocotyl. The ozone water is thus only able to disinfect the hyphae that have left the agar substrate, after which the fungus immediately begins to grow again as growth starting at the fixed substrate. A significant result of this research from a technical point of view is the control of aerobic bacteria spreading on the surface of the hyphae by means of ozone.

### Analysis of results

The powdery mildew research showed beyond a shadow of a doubt that the powdery mildew of plants can be reliably controlled or at least limited in the event of a high infection intensity. A requirement of the control treatments is that the ozone water can be conveyed to the surface of the plant in large droplets, which by their size are reminiscent of natural medium-intensity precipitation. In the testing conducted here, treatments were not performed on Saturday or Sunday on the weekend, which definitely diminished the control result. The embodiments for the control of powdery mildew are easiest to implement in a greenhouse which already has an automatic sprinkler-type system for brief sprayings of the leaves of the plants. The control of powdery mildew is also possible outdoors, for example on strawberry plants, by means of frost-protection nozzles that can be mounted on benches.

The tests regarding the disinfection of Fusarium fungus were quite unequivocal, i.e., in the case of these typical fungi which produce small conidia, both the light-coloured conidia as well as the light-coloured mycelia are completely sensitive to ozone. The most important use for the ozone water would be the disinfecting of growing spaces and substrates between growing episodes. Its use during farming is also possible in order to protect the surface of the plant from pathogens which have not yet infected the host plant. The modus operandi would be similar to that in the case of powdery mildew diseases, i.e. spraying the sites so that they are wet.

After several tests, the control of grey mould turned out to be possible after all when the mycelium and conidia are dry. This fungus is, as its name implies, already slightly discoloured. In nature, it occurs in plant parts above ground and survives in complete aridity and sunshine like the mycelium of powdery mildew, which survives thanks to the water taken from the plant by means of its haustoria. In the first laboratory tests, however, the grey mould was completely tolerant to ozone, as the mycelium and conidia of the fungus had been growing in air with a very high humidity such as on an agar medium or on stored cabbage. When the mycelium and conidia of the fungus were allowed to dry, the cells of the mycelium had sunk so as to become flat under microscopic analysis. When this growth was rinsed into water and sprayed to dry on a plastic surface or was pipetted directly amidst ozone water, the ozone water prevented the fungus superbly. This control method is based on the hypothesis that a dry yet viable fungus must absorb the ozone water, whereby the fungus dies as a consequence of the ingested ozone. Corresponding phenomena cannot occur in nature so that grey mould has not been able to develop defence mechanisms for ozone over the course of the years. The same active mechanism works with all light-coloured fungi (*Moniliaceae* fungi). This finding has a broad potential for application in the disinfecting of greenhouses and stores. Perhaps the most significant area of application is the control of storage diseases of cabbage and carrots, in particular of grey mould and *Slerotiorum sclerotiorum.* The spores of these fungi end up being transported by the wind onto the surfaces of plants in dry weather, while in the rainy seasons the spores are washed to the ground.

## Claims

1. A method for eliminating pathogenic microbes from crops, the pathogenic microbes having stages that are sensitive to ozone during their life cycle, which are dried mycelia and spores, zoospores and sensitive mycelia that are unaccustomed to light, the method including
• producing an ozone water solution (81), and
• treating the crops with the ozone water solution (66),
**characterized in that**
• in greenhouse farming
o humidity control is provided for drying the foliage before drying the pathogenic microbes and the evaporation rate is set in a manner that the drying of the water on the surfaces of the crops takes 5 - 300, most advantageously 60 - 150 minutes (62),
∘ the pathogenic spores and/or organisms on the crops are dried and placed in a resting condition and an absorbent state by the drying before the treatment with the ozone water solution (64) by the setting of the evaporation rate,
∘ the following standard time is determined for the drying time of the microbes (64), the standard time corresponding to an evaporation rate at a relative humidity of 60 - 80%, a radiation of 200 W/m² and an air flow of 2 m/s, whereby the drying time is 1 - 7 hours, most advantageously 2 - 4 hours, whereby, if there is a change in conditions and thus in the evaporation rate, a commensurate drying time is calculated to correspond to the same overall evaporation,
• or in outdoor farming
∘ the evaporation rate is monitored,
∘ one waits for conditions in which the evaporation rate is sufficient to allow treatment after the drying stage of the foliage and the pathogenic spores and/or organisms on the crops,
∘ the ozone treatment is carried out when the pathogenic spores and/or organisms are in an absorbent state during the day at a time without rain when conditions have brought about the aforementioned drying,
∘ the following standard time is determined for the drying time of the microbes (64), the standard time corresponding to an evaporation rate at a relative humidity of 60 - 80%, a radiation of 200 W/m² and an air flow of 2 m/s, whereby the drying time is 1 - 7 hours, most advantageously 2 - 4 hours, whereby, if there is a change in conditions and thus in the evaporation rate, a commensurate drying time is calculated to correspond to the same overall evaporation,
• after the drying stages the ozone water treatment (66) is carried out on the parts of the crops above ground with ozone water solution, in which the initial level of ozone concentration is 500 - 3000 ppb, advantageously 700 - 2500 ppb, with droplets in such a manner that the ozone concentration of the ozone water solution on the surface of the crop to be treated is 100 - 3000 ppb, advantageously 200 - 1000 ppb, whereby said spores and/or organisms absorb the ozone together with the sprayed water and die.

2. The method according to claim 1 for eliminating pathogenic microbes from plant parts above ground in greenhouse farming, wherein the life cycle of the microbes includes a significant stage outside the plant on plant parts above ground, **characterized in that**
• the conditions of the plant environment are monitored, said monitoring comprising at least one measurement of the evaporation rate, by means of which monitoring the conditions are regulated,
• a control unit (30) controls the apparatus for spraying the ozone water solution in accordance with an adapted program.

3. The method according to claims 1 or 2, **characterized in that** spraying nozzles (25) are used for the spraying (66) of the ozone water solution, said spraying nozzles (25) forming droplets the ozone concentration of which at the object is 10 - 75 % of its initial level in the water to be used for spraying.

4. The method according to any of claims 1 - 3 for destroying pathogenic microbes in a greenhouse or outdoors, **characterized in that** the ozone water treatment (66) is carried out at least once during germination and 0 - 6 times a day, advantageously 1 - 4 times a day, and at the very least once a week, as needed.

5. The method according to claim 4, **characterized in that** the daily allotment of the ozone treatment (66) is determined based on a defined level of risk.

6. The method according to any of claims 1 - 5 for destroying pathogenic microbes in a greenhouse or outdoors, **characterized in that** a single spraying of the ozone water solution lasts 5 - 60 seconds, advantageously 10-20 seconds.

7. The method according to any of claims 1 - 6, **characterized in that** the spraying pipe arrangement (21, 23) is emptied of the old water before the ozone water solution is conducted into the pipe arrangement.

8. The method according to any of claims 1 - 7, **characterized in that** the droplet size of the ozone water solution to be sprayed is 0.5 - 10 mm, advantageously 1 - 8 mm, on the SMD scale.

9. The method according to any of claims 1 - 8, **characterized in that** carbon dioxide is added to the water in addition to the ozone in order to improve the stability of the ozone.

10. A system for eliminating pathogens from crops in greenhouse farming, which includes
• an ozone-water solution generator (12) for producing a 100 - 3000 ppb, advantageously 200 - 1000 ppb, ozone water solution, and
• a pipe arrangement for the conveyance of the ozone water solution comprising a trunk water pipe (21) and several branch pipes (23) ending in several spraying nozzles (25) located among or above the plants,
**characterized in that**
• the system further includes observation means for monitoring the ambient conditions, comprising at least one sensor (32) for measuring temperature and one sensor (34) for measuring the saturation deficit,
• a control unit (30) adapted to control the apparatus for spraying the ozone water solution in accordance with an adapted program, producing a 5-300-minute, advantageously a 60-150-minute, evaporation period for drying the foliage and a 1-7-hour, advantageously a 2-4-hour, drying period for placing the spores in an absorbent condition, and
• spraying nozzles (25) that can be used for the spraying of the ozone water solution, which are adapted to form droplets in such a manner that the ozone concentration at the object is 10 - 75 % of its initial level.

11. The system according to claim 10, **characterized in that** the production of the ozone water includes a storage tank (16), which is connected via a pump (13) to a standard fertilizer-irrigation system via a valve (51, 52) .

12. The system according to claim 10 or 11, **characterized in that** the spraying nozzles (25) for spraying the ozone water solution are low-pressure fan nozzles.

13. The system according to claim 10 or 11, **characterized in that** the system includes a controllable drain valve (42) for draining the spraying pipe arrangement (21, 23) before the spraying of the ozone water.

14. The system according to claim 10 or 11, **characterized in that** the ozone-producing apparatus comprises, in addition to the ozonator (12), a compressor (123) and an air dryer (124) and a temporary storage tank (16) adapted to be pressureless and to ventilate to the outside of the greenhouse (101).

15. A method for eliminating pathogenic microbes from the surfaces of crop processing spaces, the pathogenic microbes having stages sensitive to ozone during their life cycle, which are dried mycelia and spores, zoospores and sensitive mycelia that are unaccustomed to light, the method including
• producing an ozone water solution (80), and
• treating the surfaces with the ozone water solution (66),
**characterized in that**
• the evaporation rate is set in such a manner that the drying of the water on the surfaces takes 5-400 minutes (62),
• the pathogenic spores and/or organisms on the surfaces are placed in a resting condition and an absorbent state by the drying before the treatment with the ozone water solution (64),
• the ozone water treatment (66) is carried out with ozone water solution, in which the initial level of ozone concentration is 500 - 3000 ppb, advantageously 700 - 2500 ppb, with droplets in such a manner that the ozone concentration of the ozone water solution on the surface to be treated is 100 - 3000 ppb, advantageously 200 - 1000 ppb, whereby said spores and/or organisms absorb the ozone and die.

## Patentansprüche

1. Verfahren zur Beseitigung von pathogenen Mikroben aus Feldfrüchten, wobei die pathogenen Mikroben über Stufen verfügen, die während ihres Lebenszyklus sensibel auf Ozon sind, bei denen es sich um getrocknete Myzele und Sporen, Zoosporen und sensitive Myzelen, die nicht an Licht gewöhnt sind, wobei das Verfahren Folgendes umfasst:
• Herstellen einer Ozon-Wasser-Lösung (81) und
• Behandeln der Feldfrüchte mit der Ozon-Wasser-Lösung (66),
**dadurch gekennzeichnet, dass**
• beim Anbau in Gewächshäusern
∘ eine Feuchtigkeitssteuerung vorhanden ist, um das Blattwerk zu trocknen, bevor die pathogenen Mikroben getrocknet werden, und die Verdunstungsrate auf eine Weise eingestellt ist, dass das Trocknen des Wassers auf der Oberfläche der Feldfrüchte 5-300, am vorteilhaftesten 60-150 Minuten dauert (62).
∘ die pathogenen Sporen bzw. Organismen auf den Feldfrüchten getrocknet werden und durch das Trocknen vor der Behandlung mit der Ozon-Wasser-Lösung (64) in eine Ruhebedingung und einen absorbierenden Zustand versetzt werden, indem die Verdunstungsrate eingestellt wird,
∘ der folgende Standardzeitraum für die Trocknungszeit der Mikroben bestimmt wird (64), wobei der Standardzeitraum einer Verdunstungsrate bei einer relativen Feuchtigkeit von 60-80 % entspricht, einer Strahlung von 200 W/m² und einem Luftstrom von 2 m/s, wobei die Trocknungszeit 1-7 Stunden beträgt, am vorteilhaftesten 2-4 Stunden, wobei, sollte eine Änderung der Bedingungen und damit der Verdunstungsrate eintreten, eine entsprechende Trocknungszeit berechnet wird, die der gleichen Gesamtverdunstung entspricht,
• oder beim Anbau im Freien
∘ die Verdunstungsrate überwacht wird,
∘ abgewartet wird, bis die Bedingungen, unter denen die Verdunstungsrate ausreichend ist, um eine Behandlung nach dem Trocknen des Blattwerks und der pathogenen Sporen bzw. Organismen auf den Feldfrüchten zu ermöglichen,
∘ die Ozon-Behandlung durchgeführt wird, wenn die pathogenen Sporen bzw. Organismen sich in einem absorbierenden Zustand im Laufe des Tages zu einem Zeitpunkt befinden, an dem es nicht regnet, und wenn Bedingungen vorlagen, die das oben erwähnte Trocknen bewirkt haben,
∘ der folgende Standardzeitraum für die Trocknungszeit der Mikroben bestimmt wird (64), wobei der Standardzeitraum einer Verdunstungsrate bei einer relativen Feuchtigkeit von 60-80 % entspricht, einer Strahlung von 200 W/m² und einem Luftstrom von 2 m/s, wobei die Trocknungszeit 1-7 Stunden beträgt, am vorteilhaftesten 2-4 Stunden, wobei, sollte eine Änderung der Bedingungen und damit der Verdunstungsrate eintreten, eine entsprechende Trocknungszeit berechnet wird, die der gleichen Gesamtverdunstung entspricht,
• nach den Trocknungsstufen die Ozon-Wasser-Behandlung (66) an den oberirdischen Teilen der Feldfrüchte mit einer Ozone-Wasser-Lösung durchgeführt wird, bei der das anfängliche Niveau der Ozon-Konzentration 500-3.000 ppb beträgt, vorteilhafterweise 700-2.500 ppb, mit Tropfen in einer Weise, dass die Ozon-Konzentration der Ozon-Wasser-Lösung auf der Oberfläche der zu behandelnden Feldfrucht 100-3.000 ppb beträgt, vorteilhafterweise 200-1.000 ppb, wobei die Sporen bzw. Organismen das Ozon gemeinsam mit dem gesprühten Wasser absorbieren und absterben.

2. Verfahren nach Anspruch 1 zur Beseitigung pathogener Mikroben von überirdischen Pflanzenteilen beim Anbau in Gewächshäusern, wobei der Lebenszyklus des Mikroben eine wesentliche Stufe außerhalb der Pflanze auf überirdischen Pflanzenteilen einschließt, **dadurch gekennzeichnet, dass**
• die Bedingungen der Pflanzenumgebung überwacht werden, und die Überwachung mindestens eine Messung der Verdunstungsrate umfasst, sodass durch die Überwachung die Bedingungen reguliert werden,
• eine Steuereinheit (30) das Gerät zum Versprühen der Ozon-Wasser-Lösung gemäß einem angepassten Programm steuert.

3. Verfahren nach Anspruch 1 oder 2, **dadurch gekennzeichnet, dass** für das Versprühen (66) der Ozon-Wasser-Lösung Sprühdüsen (25) verwendet werden, wobei die Sprühdüsen (25) Tropfen bilden, deren Ozon-Konzentration am Objekt 10-75 % des ursprünglichen Niveaus im für das Versprühen zu verwendenden Wasser enspricht.

4. Verfahren nach einem der Ansprüche 1-3 zum Zerstören pathogener Mikroben in einem Gewächshaus oder im Freien, **dadurch gekennzeichnet, dass** die Ozon-Wasser-Behandlung (66) mindestens einmal während der Keimung und 0-6 mal am Tag durchgeführt wird, vorteilhafterweise 1-4 mal pro Tag und mindestens einmal pro Woche nach Bedarf.

5. Ein Verfahren nach Anspruch 4, **dadurch gekennzeichnet, dass** die Menge der Speise (66) in Echtzeit in Verbindung mit der Auswahl festgestellt wird.

6. Verfahren nach einem der Ansprüche 1-5 zum Zerstören pathogener Mikroben in einem Gewächshaus oder im Freien, **dadurch gekennzeichnet, dass** ein einzelner Sprühvorgang der Ozon-Wasser-Lösung 5-60 Sekunden dauert, vorteilhafterweise 10-20 Sekunden.

7. Verfahren nach einem der Ansprüche 1-6, **dadurch gekennzeichnet, dass** das alte Wasser aus der Sprühschlauchvorrichtung (21, 23) geleert wird, bevor die Ozon-Wasser-Lösung in die Sprühschlauchvorrichtung eingefüllt wird.

8. Verfahren nach einem der Ansprüche 1-7, **dadurch gekennzeichnet, dass** die Tropfengröße der zu versprühenden Ozon-Wasser-Lösung 0,5-10 mm beträgt, vorteilhafterweise 1-8 mm auf der SMD-Skala.

9. Verfahren nach einem der Ansprüche 1-8, **dadurch gekennzeichnet, dass** dem Wasser zusätzlich zu dem Ozon Kohlendioxid zugesetzt wird, um die Stabilität des Ozons zu verbessern.

10. System zur Beseitigung von Pathogenen aus Feldfrüchten beim Anbau in Gewächshäusern, welches Folgendes einschließt:
• einen Ozon-Wasser-Lösungs-Generator (12) zur Herstellung einer Ozon-Wasser-Lösung mit 100-3.000 ppb, vorteilhafterweise 200-1.000 ppb und
• eine Schlauchvorrichtung für die Beförderung der Ozon-Wasser-Lösung, die eine Stammwasserleitung (21) und mehrere Zweigleistungen (23) umfasst, die in mehreren Sprühdüsen (25) zwischen oder über den Pflanzen enden,
**dadurch gekennzeichnet, dass**
• das System weiterhin Beobachtungsmittel zur Überwachung der Umgebungsbedingungen einschließt, umfassend mindestens einen Sensor (32) zur Temperaturmessung und einen Sensor (34) zur Messung des Sättigungsdefizits,
• eine Steuereinheit (30), die eingestellt ist, um die Vorrichtung zum Versprühen der Ozon-Wasser-Lösung gemäß einem angepassten Programm zu steuern, und eine 5-300 Minuten lange, vorteilhafterweise eine 60-150 Minuten lange Verdunstungszeit zum Trocknen des Blattwerks zu erzeugen und einen 1-7-stündigen, vorteilhafterweise einen 2-4-stündeigen Trocknungszeitraum zu erzeugen, um die Sporen in einen absorbierenden Zustand zu versetzen, und
• Sprühdüsen (25) die für das Sprühen der Ozon-Wasser-Lösung verwendet werden kann, die eingestellt wind, um dergestalt Tropfen zu bilden, dass die Ozon-Konzentration am Objekt 10-75 % des ursprünglichen Niveaus beträgt.

11. System nach Anspruch 10, **dadurch gekennzeichnet, dass** die Herstellung des Ozon-Wassers einen Speichertank (16) einschließt, der über eine Pumpe (13) mit einem Standard-Dünge-Bewässerungssystem mittels eines Ventils (51, 52) verbunden ist.

12. System nach Anspruch 10 oder 11, **dadurch gekennzeichnet, dass** die Sprühdüsen (25) zum Versprühen der Ozon-Wasser-Lösung Niederdruck-Strahldüsen sind.

13. System nach Anspruch 10 oder 11, **dadurch gekennzeichnet, dass** das System ein steuerbares Abflussventil (42) zum Ablassen der Sprühschlauchvorreichtung (21, 23) vor dem Versprühen des Ozon-Wassers einschließt.

14. System nach Anspruch 10 oder 11, **dadurch gekennzeichnet, dass** die Ozon erzeugende Vorrichtung zusätzlich zu dem Ozonator (12) einen Kompressor (123) und einen Lufttrockner (124) umfasst sowie einen Zwischenspeichertank (16), der eingestellt ist, drucklos zu sein und aus dem Gewächshaus hinaus zu lüften (101).

15. Verfahren zur Beseitigung von pathogenen Mikroben von der Oberfläche von Feldfruchtverarbeitungsräumen, wobei die pathogenen Mikroben über Stufen verfügen, die während ihres Lebenszyklus sensibel auf Ozon sind, bei denen es sich um getrocknete Myzele und Sporen, Zoosporen und sensitive Myzelen, die nicht an Licht gewöhnt sind, handelt, wobei das Verfahren Folgendes umfasst:
• Herstellen einer Ozon-Wasser-Lösung (80) und
• Behandeln der Oberflächen mit der Ozon-Wasser-Lösung (66),
**dadurch gekennzeichnet, dass**
• die Verdunstungsrate auf eine Weise eingestellt ist, dass das Trocknen des Wassers auf der Oberfläche 5-400 dauert (62),
• die pathogenen Sporen bzw. Organismen auf den Oberflächen durch das Trocknen vor der Behandlung mit der Ozon-Wasser-Lösung (64) in eine Ruhebedingung und einen absorbierenden Zustand versetzt werden,
• die Ozon-Wasser-Behandlung (66) mit einer Ozon-Wasser-Lösung durchgeführt wird, bei der das anfängliche Niveau der Ozon-Konzentration 500-3.000 ppb beträgt, vorteilhafterweise 700-2.500 ppb, mit Tropfen in einer Weise, dass die Ozon-Konzentration der Ozon-Wasser-Lösung auf der zu behandelnden Oberfläche 100-3.000 ppb beträgt, vorteilhafterweise 200-1.000 ppb, wobei die Sporen bzw. Organismen das Ozon gemeinsam mit dem gesprühten Wasser absorbieren und absterben.

## Revendications

1. Un procédé pour éliminer des microbes pathogènes des cultures, les microbes pathogènes présentant des stades sensibles à l'ozone pendant leur cycle de vie, qui sont des mycéliums et des spores séchés, des zoospores et des mycéliums sensibles qui ne sont pas habitués à la lumière, le procédé consistant
• à produire une solution d'eau ozonisée (81) et
• à traiter les cultures avec la solution d'eau ozonisée (66),
**caractérisé en ce que**
• dans la serriculture
o un contrôle de l'humidité est prévu pour le séchage du feuillage avant le séchage des microbes pathogènes, et le taux d'évaporation est défini de sorte que le séchage de l'eau sur les surfaces des cultures prend entre 5 et 300 minutes, plus avantageusement entre 60 et 150 minutes (62),
∘ les organismes et/ou spores pathogènes des cultures sont séchés et placés dans une condition de repos et dans un état absorbant par le séchage avant le traitement avec la solution d'eau ozonisée (64) selon la définition du taux d'évaporation,
∘ le temps standard suivant est déterminé pour le temps de séchage des microbes (64), le temps standard correspondant à un taux d'évaporation avec une humidité relative de 60 à 80 %, un rayonnement de 200 W/m² et un débit d'air de 2 m/s, le temps de séchage allant de 1 à 7 heures, plus avantageusement de 2 à 4 heures, et en cas de changement des conditions et donc du taux d'évaporation, un temps de séchage proportionnel est calculé pour correspondre à la même évaporation globale,
• ou dans l'agriculture de plein air
∘ le taux d'évaporation est surveillé,
∘ on attend les conditions dans lesquelles le taux d'évaporation est suffisant pour permettre le traitement après le stade de séchage du feuillage et des organismes et/ou spores pathogènes des cultures,
∘ le traitement à l'ozone est effectué quand les organismes et/ou spores pathogènes sont dans un état absorbant durant la journée à un moment sans pluie où les conditions ont provoqué le séchage précité,
∘ le temps standard suivant est déterminé pour le temps de séchage des microbes (64), le temps standard correspondant à un taux d'évaporation avec une humidité relative de 60 à 80 %, un rayonnement de 200 W/m² et un débit d'air de 2 m/s, le temps de séchage allant de 1 à 7 heures, plus avantageusement de 2 à 4 heures, et en cas de changement des conditions et donc du taux d'évaporation, un temps de séchage proportionnel est calculé pour correspondre à la même évaporation globale,
• après les stades de séchage, le traitement à l'eau ozonisée (66) est effectué sur les parties des cultures au-dessus du sol avec la solution d'eau ozonisée, dans laquelle le niveau initial de concentration en ozone est de 500 à 3 000 ppb, avantageusement de 700 à 2 500 ppb, avec des gouttelettes de sorte que la concentration en ozone de la solution d'eau ozonisée sur la surface de la culture à traiter est de 100 à 3 000 ppb, avantageusement de 200 à 1 000 ppb, moyennant quoi lesdits spores et/ou organismes absorbent l'ozone conjointement avec l'eau pulvérisée et meurent.

2. Le procédé conformément à la revendication 1 pour éliminer les microbes pathogènes des parties de plantes au-dessus du sol dans la serriculture, où le cycle de vie des microbes présente un stade important en dehors des plantes sur les parties de plantes au-dessus du sol, **caractérisé en ce que**
• les conditions de l'environnement des plantes sont surveillées, ladite surveillance incluant au moins une mesure du taux d'évaporation, ce qui permet de réguler les conditions,
• une unité de commande (30) contrôle l'équipement pulvérisant la solution d'eau ozonisée conformément à un programme adéquat.

3. Le procédé conformément à la revendication 1 ou 2,
**caractérisé en ce que** les gicleurs (25) sont utilisés pour la pulvérisation (66) de la solution d'eau ozonisée, lesdits gicleurs (25) formant des gouttelettes dont la concentration d'ozone sur l'objet est de 10 à 75 % de son niveau initial dans l'eau servant à la pulvérisation.

4. Le procédé conformément à l'une quelconque des revendications 1 à 3 pour la destruction des microbes pathogènes en serre ou en plein air, **caractérisé en ce que** le traitement à l'eau ozonisée (66) est réalisé au moins une fois au cours de la germination, et entre 0 et 6 fois par jour, avantageusement entre 1 et 4 fois par jour, et au minimum une fois par semaine, selon les besoins.

5. Le procédé conformément à la revendication 4,
**caractérisé en ce que** la quantité quotidienne de traitement à l'ozone (66) est déterminée en fonction d'un niveau défini de risque.

6. Le procédé conformément à l'une quelconque des revendications 1 à 5 pour la destruction des microbes pathogènes en serre ou en plein air, **caractérisé en ce qu'**une seule pulvérisation de la solution d'eau ozonisée dure entre 5 et 60 secondes, avantageusement entre 10 et 20 secondes.

7. Le procédé conformément à l'une quelconque des revendications 1 à 6, **caractérisé en ce que** le dispositif composé de conduites servant à la pulvérisation (21, 23) est vidé de l'ancienne eau avant l'introduction de la solution d'eau ozonisée.

8. Le procédé conformément à l'une quelconque des revendications 1 à 7, **caractérisé en ce que** la taille des gouttelettes de la solution d'eau ozonisée à pulvériser est comprise entre 0,5 et 10 mm, avantageusement entre 1 et 8 mm, selon l'échelle du diamètre de Sauter.

9. Le procédé conformément à l'une quelconque des revendications 1 à 8, **caractérisé en ce que** le dioxyde de carbone est ajouté à l'eau en plus de l'ozone afin d'améliorer la stabilité de l'ozone.

10. Un système pour éliminer les pathogènes des cultures dans la serriculture, qui inclut
• un générateur de solution d'eau ozonisée (12) pour produire 100 à 3 000 ppb, avantageusement 200 à 1 000 ppb, de solution d'eau ozonisée, et
• un dispositif composé de conduites pour acheminer la solution d'eau ozonisée comprenant une conduite principale d'adduction d'eau (21) et plusieurs conduites de dérivation (23) avec plusieurs gicleurs à leur extrémité (25) situés parmi les plantes ou au-dessus de celles-ci,
**caractérisé en ce que**
• le système comprend en outre des moyens d'observation pour surveiller les conditions ambiantes, incluant au moins un capteur (32) pour mesurer la température et un capteur (34) pour mesurer le déficit de saturation,
• une unité de commande (30) appropriée pour contrôler l'équipement pulvérisant la solution d'eau ozonisée conformément à un programme adéquat, entraînant une période d'évaporation de 5 à 300 minutes, avantageusement de 60 à 150 minutes, pour sécher le feuillage et une période de séchage de 1 à 7 heures, avantageusement de 2 à 4 heures, pour placer les spores dans un état absorbant, et
• les gicleurs (25) qui peuvent servir à pulvériser la solution d'eau ozonisée permettent de former des gouttelettes de sorte que la concentration d'ozone sur l'objet est de 10 à 75 % de son niveau initial.

11. Le système conformément à la revendication 10, **caractérisé en ce que** la production d'eau ozonisée comprend un réservoir de stockage (16), qui est raccordé par le biais d'une pompe (13) à un système classique d'irrigation et de fertilisation au moyen d'une valve (51, 52).

12. Le système conformément à la revendication 10 ou 11, **caractérisé en ce que** les gicleurs (25) pour pulvériser la solution d'eau ozonisée sont des buses à jet plat à basse pression.

13. Le système conformément à la revendication 10 ou 11, **caractérisé en ce que** le système comprend une soupape de vidange manœuvrable (42) pour vidanger le dispositif composé de conduites servant à la pulvérisation (21, 23) avant de pulvériser l'eau ozonisée.

14. Le système conformément à la revendication 10 ou 11, **caractérisé en ce que** l'équipement de production d'ozone comprend, en plus de l'ozoniseur (12), un compresseur (123) et un sécheur d'air (124) ainsi qu'un réservoir de stockage temporaire (16) adapté pour être sans pression et pour ventiler vers l'extérieur de la serre (101).

15. Un procédé pour éliminer des microbes pathogènes des surfaces des espaces de traitement des cultures, les microbes pathogènes présentant des stades sensibles à l'ozone pendant leur cycle de vie, qui sont des mycéliums et des spores séchés, des zoospores et des mycéliums sensibles qui ne sont pas habitués à la lumière, le procédé consistant
• à produire une solution d'eau ozonisée (80) et
• à traiter les cultures avec la solution d'eau ozonisée (66),
**caractérisé en ce que**
• le taux d'évaporation est défini de sorte que le séchage de l'eau sur les surfaces prend entre 5 et 400 minutes (62),
• les organismes et/ou spores pathogènes sur les surfaces sont placés dans une condition de repos et dans un état absorbant par le séchage avant le traitement avec la solution d'eau ozonisée (64),
• le traitement à l'eau ozonisée (66) est effectué avec la solution d'eau ozonisée, dans laquelle le niveau initial de concentration en ozone est de 500 à 3 000 ppb, avantageusement de 700 à 2 500 ppb, avec des gouttelettes de sorte que la concentration en ozone de la solution d'eau ozonisée sur la surface de la culture à traiter est de 100 à 3 000 ppb, avantageusement de 200 à 1 000 ppb, moyennant quoi lesdits spores et/ou organismes absorbent l'ozone et meurent.
